Europäisches Patentamt

(19) European Patent Office

Office européen des brevets

(11) Publication number: **0 070 584**
**B1**

(12) **EUROPEAN PATENT SPECIFICATION**

(45) Date of publication of patent specification: **11.09.85**

(21) Application number: **82200801.7**

(22) Date of filing: **29.06.82**

(51) Int. Cl.⁴: **A 41 B 13/02**

(54) **Disposable diaper having elasticized flaps provided with leakage resistant portions.**

(30) Priority: **17.07.81 CA 381995**

(43) Date of publication of application:
**26.01.83 Bulletin 83/04**

(45) Publication of the grant of the patent:
**11.09.85 Bulletin 85/37**

(84) Designated Contracting States:
**AT BE CH DE FR IT LI LU NL SE**

(56) References cited:
**CH-A- 587 030**
**CH-A- 608 699**
**GB-A-2 063 677**
**US-A-4 050 462**
**US-A-4 090 515**
**US-A-4 094 319**
**US-A-4 108 179**
**US-A-4 259 958**

(73) Proprietor: **THE PROCTER & GAMBLE COMPANY**
**301 East Sixth Street**
**Cincinnati Ohio 45202 (US)**

(72) Inventor: **Aziz, Mohammed Iqbal**
**3710 York lane**
**Cincinnati Ohio 45215 (US)**
Inventor: **Blaney, Ted Lee**
**7230 Jerry Drive**
**West Chester Ohio 45069 (US)**

(74) Representative: **Gibson, Tony Nicholas et al**
**Procter & Gamble (NTC) Limited Whitley Road**
**Longbenton Newcastle upon Tyne NE12 9TS**
**(GB)**

Courier Press, Leamington Spa, England.

## Description

This invention relates to disposable absorbent articles in general and more particularly relates to disposable diapers and the like. Still more particularly, this invention relates to disposable diapers having a multiplicity of flaps along the longitudinal sides of the absorbent core in which the flaps have an elastically contractible distal edge, a fixed edge and a non-wicking, non-absorbent, liquid impermeable leakage resistant member interposed between the distal and fixed edges on the liquid contacting surface.

Disposable absorbent articles are well known and have many uses. For example, disposable diapers are intended to absorb and contain urine; bandages are intended to absorb and contain blood and other body exudates; while catamenial pads are intended to absorb and retain menstrual fluids. In each instance, the disposable absorbent article absorbs and retains a liquid, thereby preventing that liquid from soiling, wetting, or otherwise contaminating the vicinity surrounding the point of liquid discharge. For example, U.S. Patent Re. 26,151 which issued on January 31, 1967 to R. C. Duncan et al. entitled "Disposable Diaper" teaches a disposable diaper intended to aborb urine and prevent the wetting of the wearer's clothing.

Disposable absorbent articles should perform without leaking and several concepts have been proposed to improve the liquid containment characteristics of disposable absorbent articles such as disposable diapers. U.S. Patent 3,999,548 entitled "Disposable Diaper Having Fluid Trap" which issued to J. Hernandez on December 28, 1976 teaches that the liquid containment characteristics of a diaper can be improved by securing sealing strips of waterproof material to the face sheet of the diaper. Alternatively, U.S. Patent 3,860,003 entitled "Contractible Side Portions For A Disposable Diaper" which issued to K. B. Buell on January 14, 1975 and U.S. Patent 4,050,462 Entitled "Disposable Diaper With Elastically Constricted Crotch Section" which issued to L. S. Woon et al. on September 7, 1977 both teach a concept for reducing liquid leakage which involves providing an elastic member in a disposable diaper. The elastic member is positioned so that when the diaper is worn, the diaper is drawn snugly about the legs of the wearer. The elastic causes the diaper to form a seal about the leg of the wearer thereby preventing liquid from leaking out of the diaper.

The disposable absorbent articles of the prior art lack the aspects of the present invention whereby an improvement in the liquid containment characteristics is obtained by providing elastically contractible flaps along the longitudinal sides of the article which flaps have a leakage resistant portion which is non-wicking, non-absorbent, and liquid impermeable.

It is therefore an object of the present invention to provide a disposable absorbent article having improved liquid containment characteristics.

A further object of the present invention is to provide a disposable absorbent article having elastically contractible flaps provided with a leakage resistant portion which is non-wicking, non-absorbent, and liquid impermeable.

## Summary of the Invention

According to the present invention, a disposable diaper comprising:

an outer covering layer comprising a topsheet portion and a backsheet portion, said sheet portion being joined together with said backsheet portion;

an absorbent core means for absorbing liquid, said absorbent core means having a peripheral edge comprising a first longitudinal side edge and a second longitudinal side edge a multiplicity of flaps each of said flaps having a fixed edge, a distal edge in spaced relation to said fixed edge, and having a liquid contacting surface, the longitudinal sides of which are bounded by said fixed and said distal edges, one of said flaps having said fixed edge connected to said outer covering layer at said first longitudinal side edge of said absorbent core means and one of said flaps having said fixed edge connected to said outer covering layer at said second longitudinal side edge the distal edge of each of said flaps being displaced from said absorbent core means; and

an elastic element affixed to each of said flaps, wherein said elastic element is operatively associated with the distal edge of each of said flaps and in that each of said flaps has a leakage resistant portion on said liquid contacting surface interposed between said fixed edge and said distal edge, said leakage resistant portion being non-absorbent, and liquid impermeable.

A leakage resistant portion which is non-wicking, non-absorbent and liquid impermeable is interposed on the liquid contacting surface of the flaps, between the fixed and distal edges. Thus, in order for liquid to flow or wick from the point of discharge to the distal edge where liquid leakage about the legs can occur it is necessary for the liquid to move across the leakage resistant portion. The leakage resistant portion is non-wicking and non-absorbent which characteristics prevent liquid from reaching the distal edge of the flap from which leakage from the diaper can occur.

## Brief Description of the Drawings.

Figure 1 is a perspective view of a disposable diaper which incorporates the present invention and which is Z-folded and ready to be placed on an infant.

Figure 2 is a partially cut away perspective view of the disposable diaper of Figure 1 prior to being Z-folded and contracted.

Figure 3 is a cross-sectional view of the diaper of Figure 2 taken along line 3—3.

Figure 4 is a cross-sectional view of an alternatively preferred embodiment of the present invention taken along a line corresponding to line 3—3.

Figure 5 is a cross-sectional view of an alternatively preferred embodiment of the present invention taken along a line corresponding to line 3—3.

Figure 6 is a cross-sectional view of an alternatively preferred embodiment of the present invention taken along a line corresponding to line 3—3.

Description of a Preferred Embodiment

Referring now to the figures, there is shown a preferred embodiment of the present invention as it would be used in a dispoable absorbent article and, in particular, as it would be used in a disposable diaper. As used herein, the term disposable absorbent article refers to articles which absorb and contain liquid, and more specifically refers to articles which are placed against or in proximity to the human body to absorb and contain the various liquids discharged therefrom (e.g., blood, menses, urine, etc.), and further which articles are intended to be discarded after a single use (i.e., they are not intended to be laundered or otherwise restored and reused). A "diaper" is a garment generally worn by infants or incontinent persons, which is drawn up between the legs and fastened about the waist of the user. It should be understood, however, that the present invention is also applicable for use in other disposable absorbent articles such as bandages, catamenial pads, and the like.

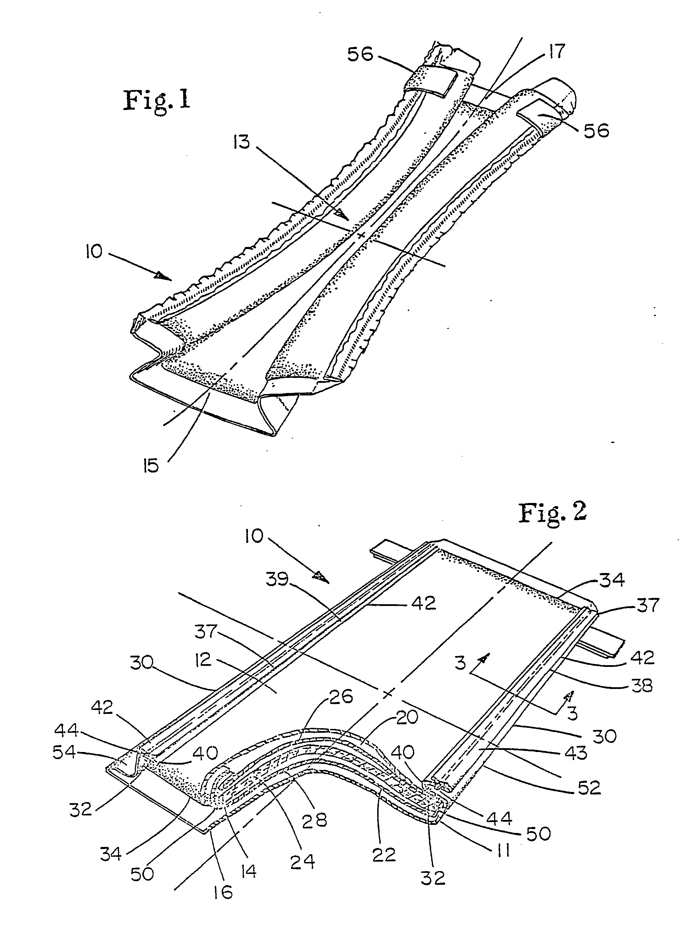

Figure 1 is a perspective view of a diaper 10 having a Z-folded side configuration and incorporating the features of the present invention. As shown in Figure 1 the diaper 10 is in condition for placement on a wearer. In general, the crotch portion 13 of the diaper 10 is placed between the wearer's legs and the front and back waist portions 15 and 17 respectively, are joined together by adhesive tapes 56 so as to encircle the wearer's waist and to hold the diaper 10 in place. While the present invention will be described with reference to a diaper having a Z-folded side configuration, it should be understood that diaper configurations and constructions other than those specifically described, such as C-folded and unfolded side configurations, may also incorporate the features of the present invention.

To simplify the description of the present invention the diaper 10 of Figure 1 is shown in Figure 2 in a partially cut-away perspective view prior to its being Z-folded, contracted, and placed on the diaper wearer. As seen in Figure 2, a preferred disposable diaper 10 basically comprises an outer covering layer 11 and an absorbent core 14. While the outer covering layer 11 and the absorbent core 14 may be assembled in a variety of well known configurations, such as are described generally in U.S. Patent Re. 26,151 entitled "Diaper" which issued to R. C. Duncan et al. on January 31, 1967, and in U.S. Patent 3,860,003 entitled "Contractible Side Portions For Disposable Diaper" which issued to K. B. Buell on January 14, 1975, a preferred construction of the diaper 10 will now be described.

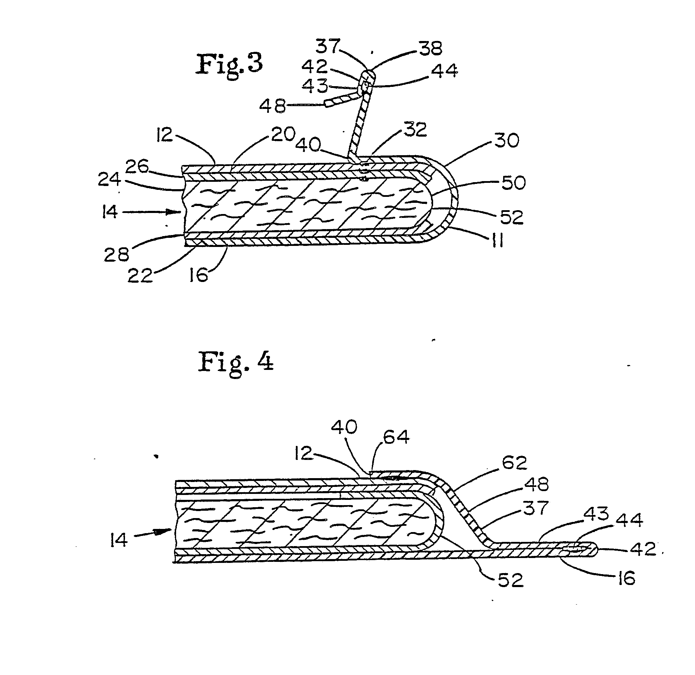

As can be seen in Figures 2 and 3, a preferred outer covering layer 11 encases and contains the absorbent core 14 and preferably has a topsheet portion 12 and a backsheet portion 16 which are joined together in any suitable matter. As used herein, the term "joined" encompasses configurations whereby the topsheet portion 12 is directly joined to the backsheet portion 16 by affixing the topsheet portion 12 directly to the backsheet portion 16 and configurations whereby the topsheet portion 12 is indirectly joined to the backsheet portion 16 by affixing the topsheet portion 12 to intermediate members which in turn is affixed to the backsheet portion 16. In the preferred embodiment of Figures 2 and 3, the topsheet portion 12 and the backsheet portion 16 are joined directly to each other.

The absorbent core 14 may be any means which is generally compressible, conformable, non-irritating to the wearer's skin, and which is capable of absorbing and retaining liquids. A preferred absorbent core 14 has first and second opposed faces 20 and 22 respectively, and comprises an absorbent layer 24 and first and second tissue layers 26 and 28 respectively. The first and second tissue layers 26 and 28 overlay the major surfaces of the absorbent layer 24 to form first and second opposed faces 20 and 22. The outer periphery of the absorbent core 14 forms a peripheral edge 50 having first and second longitudinal side edges 52 and 54 respectively.

The absorbent layer 24 may be manufactured in a wide variety of sizes and shapes (e.g., rectangular, hourglass, etc.) and from a wide variety of liquid absorbent materials commonly used in disposable absorbent articles, such as comminuted wood pulp which is generally referred to as absorbent fluff. Other liquid absorbing materials can also be used for the absorbent layer 24, such as a multiplicity of plies of crepe, cellulose wadding, absorbent foams or sponges, or any equivalent material. Further, the size and absorbent capacity of the absorbent layer 24 may be varied to accommodate wearers ranging from infants to adults. The preferred embodiment illustrated in Figures 2 and 3 has a rectangular absorbent layer 24 and is intended for infants of from 12 pounds to 23 pounds (5 kilograms to 10 kilograms). The absorbent layer 24 is, therefore, absorbent fluff approximately 12 inches (31.8 centimeters) wide by 16 inches (40.6 centimeters) long having an absorbent capacity of from 8 to 16 grams of water per gram of absorbent. Accordingly, the absorbent fluff used in the preferred embodiment shown in Figures 2 and 3 weighs approximately from 30 to 56 grams. It should be understood, however, that the size, shape, and total absorbent capacity of the absorbent core 14 may be varied to accommodate wearers ranging from infants to adults. Therefore, other dimensions and even other shapes (e.g., hourglass) may also be used for the absorbent core 14.

The tissue layers 26 and 28 improve the tensile strength of the absorbent core 14 and reduce the tendency of the absorbent layer 24 to split, lump or ball when wetted. The tissue layers 26 and 28 also help to improve lateral wicking of absorbed liquids, thereby providing a more even distribution of liquids throughout the absorbent core 14.

While a number of materials and manufacturing techniques may be used to manufacture the tissue layers 26 and 28, satisfactory results have been obtained with sheets of tissue paper having a basis weight of from 10 pounds per 3,000 square feet (16 grams per square meter) and having an air permeability of 100 cubic feet per minute per square foot (30.5 cubic meters per minute per square meter) over a $\frac{1}{2}$ inch (12.8 millimeter) water pressure drop. While the tissue layers 26 and 28 are preferably coterminous with the absorbent layer 24, they may have different dimensions, a different configuration, or they may be omitted entirely.

The second tissue layer 28 is superimposed on the backsheet portion 16 and is preferably attached thereto by attachment means (not shown) such as those well known in the art. For example, the absorbent core 14 can be secured to the backsheet portion 16 by a uniform continuous layer of adhesive, a patterned layer of adhesive, or an array of separate lines or spots of adhesive. An adhesive which has been found to be satisfactory is manufactured by Eastman Chemical Products Company of Kingsport, Tennessee and marketed under the tradename Eastobond A-3.

The backsheet portion 16 is impervious to liquids and is preferably manufactured from a thin plastic film, although other flexible liquid impervious materials may also be used. The backsheet portion 16 prevents the liquids contained in the absorbent core 14 from wetting articles which contact the diaper, such as bedsheets and undergarments. Polyethylene films having a thickness of from 0.0005 to 0.002 inches (0.0012 to 0.0051 centimeters) have been used for the backsheet portion 16 in a preferred embodiment with satisfactory results. As used herein, the term "flexible" refers to materials which are compliant and which will readily conform to the general shape and contours of the human body. A suitable polyethylene film is manufactured by Monsanto Chemical Company and marketed in the trade as film No. 8020.

The topsheet portion 12 is compliant, soft feeling, and non-irritating to the wearer's skin. Further, the topsheet portion 12 is liquid permeable, permitting liquids to readily penetrate through its thickness. A suitable topsheet 12 may be manufactured from a wide range of materials such as porous foams, apertured plastic films, natural fibers (e.g., wood or cotton fibers), synthetic fibers (e.g., polyester or polypropylene fibers) or from a combination of natural and synthetic fibers and prevents the wearer of the diaper 10 from contacting the absorbent core 14.

A particularly preferred topsheet portion 12 comprises by weight 65 percent staple length polyester fibers having a denier of 1.5, such as Kodel-type 411 polyester fibers marketed by Tennessee Eastman Corporation of Kingsport, Tennessee; 15 percent staple length crimped rayon fibers having a dernier of 1.5; and 20 percent acrylic copolymer binder such as Celanese CPE 8335 marketed by Celanese Corporation of Charlotte, North Carolina. As used herein, the term "staple length fibers" refers to those fibers having a length of at least $\frac{5}{8}$ inches (15.9 millimeters).

Clearly, there are a number of manufacturing techniques which may be utilized to manufacture the topsheet portion 12. For example, the topsheet portion 12 may be woven, non-woven, spun-bonded, carded or the like. A preferred topsheet portion 12 is carded, saturated with a binder solution, dried and cured by means well known to those skilled in the art. Preferably, the topsheet portion 12 has a basis weight range of from 21.5 to 26.3 g/m². A preferred topsheet portion 12 is further characterized by a minimum wet tensile strength of at least 400 grams per centimeter in a machine direction and at least 55 grams per centimeter in a cross machine direction.

The backsheet portion 16 is superimposed on the second opposed face 22 of the absorbent core 14 and has dimensions generally larger than those of the absorbent core 14. The topsheet portion 12 is superimposed on first opposed face 20 of the absorbent core 14 and is coterminous with the first and second longitudinal side edges 52 and 54, respectively, but is longer than the absorbent core 14. The longitudinal margins 30 of the backsheet portion 16 are folded onto and affixed to the topsheet portion 12 along the longitudinal seams 32 in any suitable manner such as by the use of adhesives. A suitable adhesive is manufactured by National Starch Corporation of Bridgewater, New Jersey and marketed under the tradename Instant Lock 34-2933, although other adhesives as are well known may also be used. As best seen in Figure 2, transverse edges 34 of the topsheet portion 12 are folded onto the second opposed face 22 of the absorbent core 14 and affixed to the backsheet portion 16 along transverse seams (not shown). The absorbent core 14 is, therefore, encased within the outer covering layer 11 which comprises the backsheet portion 16 and the topsheet portion 12, the backsheet portion 16, and the absorbent core 14 may be assembled into a disposable diaper is given in the hereinbefore referenced U.S. Patent Re. 26,151 entitled "Disposable Diaper" which issued to R. C. Duncan et al. on January 31, 1967.

Liquid discharged onto the diaper 10 while it is being worn will tend to be distributed throughout the diaper 10. As a result of the liquid distribution some of the liquid will move toward segments of the diaper 10 from which leakage can occur. The location of these segments and the specific means by which leakage occurs will depend on the particular construction used for the diaper 10. In general, however, liquid leakage frequently occurs at those segments of the diaper 10 which are fitted about the legs of the diaper wearer.

The figures illustrate a preferred embodiment and alternatively preferred embodiments of the present invention in which liquid leakage around the legs of the diaper wearer is to be reduced. Accordingly, the diaper 10 illustrated in Figures 2 and 3 is provided with a multiplicity of flaps 37 positioned so as to encircle the legs of the wearer when the diaper is worn. While it is not essential that the legs be completely encircled, the preferred embodiment shown in Figure 2 has flaps 37 traversing the entire length of the diaper 10.

The flaps 37 are thin, flexible bands having a fixed edge 40, a distal edge 42, and a liquid contacting surface 43. The fixed edge 40 and the distal edge 42 are in spaced relation to each other and define the width of the flaps 37. The fixed and distal edges 40 and 42 may be parallel, non-parallel, rectilinear or curvilinear. Preferably, however, the distal and fixed edges 42 and 40 are parallel and rectilinear thereby imparting a uniform width to the flaps 37. The liquid contacting surface 43 is the major surface the longitudinal sides of which are bounded by the fixed and distal edges 40 and 42 and across which surface urine (as hereinafter defined) must flow to move from the fixed edge 40 to the distal edge 42.

The preferred diaper 10 illustrated in Figure 2 is provided with a first flap 38 and with a second flap 39 each having a fixed edge 40 connected to the outer covering layer 11 at the first longitudinal side edge 52 and at the second longitudinal side edge 54 respectively. The term "connected" includes any means for affixing the flaps 37 to the outer covering layer 11 and encompasses means whereby the flaps 37 are made integral with the outer covering layer 11 (i.e., the flaps 37 are a separate element affixed to the outer covering layer 11) and means whereby the flaps 37 are unitary with the outer covering layer 11 (i.e., the flaps 27 have at least one continuous and undivided element in common with the outer covering layer 11). In the preferred diaper construction shown in Figures 2 and 3, the flaps 37 are connected to the covering layer 11 adjacent to the first and second longitudinal side edges 52 and 54 of the absorbent core 14 so that when the diaper is worn the flaps 37 will encircle the legs of the wearer. If other diaper constructions are used other placements of the flap 37 may be necessary.

The distal edge 42 is elastically contractible having a stretched length (i.e., a length when subjected to a tensile force sufficient to overcome the contractive forces of the elastic element 44) which is at least 110% of its contracted length (i.e., the length of the distal edge 42 when no tensile forces are applied and the contractive forces of the elastic element 44 are allowed to contract the distal edge 42). Thus, the distal edge 42 is the line spaced farthest from the fixed edge 40 (which distance is measured along the liquid contacting surface 43 of the flaps 37) along which elastic contraction occurs.

The distal edge 42 is preferably displaced from the absorbent core 14 a distance sufficient to permit the flaps 37, which are flexible, to be gathered about the leads of the wearer without major gaps being formed between the flaps 37 and the wearer's legs. As used herein, the term "displaced" includes distal edges 42 which are displaceable from the absorbent core 14. In other words, if the distal edge 42 may assume more than one position relative to the absorbent core 14 with equal facility then the distal edge 42 is displaceable and included in the term "displaced" even though the distal edge 42 may assume a position adjacent to the absorbent core 14 at some times. The distance from the distal edge 42 to the absorbent core 14 is measured along a line drawn from the distal edge 42 to the closest part of the absorbent core 14 when the distal edge 42 is positioned so as to be spaced from the absorbent core 14 as far as possible. Preferably, the distal edge 42 will be displaced from the absorbent core 14 a distance of at least 0.25 inches (6.3 mm). Most preferably the distal edge 42 will be displaced at least 0.50 inches (13 mm) and still more preferably at least 0.75 inches (19 mm).

While the flaps 37 may be manufactured from an elastic material or otherwise made to be elastically contractible the required elasticity is preferably provided by an elastic element 44. The elastic element 44 is affixed to the flaps 37 in an elastically contractible condition so that in a normally unrestrained configuration, the elastic element 44 effectively contracts or gathers the flap material adjacent to the elastic element 44. The placement of the elastic element 44 will determine the location of the distal edge 42 as hereinbefore described. The elastic element 44 may be affixed to the flaps 37 in an elastically contractible condition in at least two ways. For example, the elastic element 44 may be stretched to its stretched condition and fixed to the flaps 37 while the flaps 37 are in an uncontracted condition. Alternatively, the flaps 37 may be contracted, for example, by pleating, and the elastic element 44 fixed to the contracted flaps 37 while the elastic element 44 is in its relaxed or unstretched condition.

The elastic element 44 preferably develops a skin contact pressure in use of from 0.1 to 2.5 pounds per square inch (0.7 to 17 kpa). A skin contact pressure within that range is acceptable to provide continued contact of the flaps 37 with the infant's thigh without exerting a pressure which detrimentally indents or marks the skin. To provide the proper skin contact pressure, the elastic element 44 will preferably have a contractional force in its stretched condition in the range of from 10 to 200 grams and most preferably in the range of from 20 to 100 grams. The elastic element 44 should provide such a contractional force and thus establish its stretched condition at an elongation from its relaxed state in the range of from 50 to 400 percent and most preferably in the range of from 125 to 300 percent.

One elastic member 44 which has been found to work well is an elastic tape having a cross-section of 0.18 mm by 1.52 mm and made from natural rubber which is available from East Hampton Rubber Company and identified by them as L-1900 Rubber Compound. The elastic tape produces a contractual force of 50 grams when stretched 150 percent from its relaxed condition. The rubber tape was used as the elastic element 44 in the preferred embodiment of Figures 2 and 3 and was stretched 150 percent from its relaxed condition to place it in its stretched condition, i.e., its maximum stretch length as allowed by the materials used for the flaps 37 when attached to the flaps 37, and has a tension therewithin of 50 grams.

The elastic element 44, as shown in Figures 2 and 3, is operatively associated with the flaps 37 by securing it to the flaps 37 with an elastic attachment means (not shown). The elastic attachment means should be flexible and of sufficient adhesiveness to hold the elastic element 44 in its stretched condition substantially indefinitely. One material which has worked as a flexible elastic attachment means is hot melt adhesives such as marketed by Findley Adhesives, Inc., Elm Grove, Wisconsin, under the tradename Findley Adhesives 691-336. A more detailed description of how the elastic element 44 may be positioned and secured to the diaper 10 is given in the hereinbefore referenced U.S. Patent 3,860,003 entitled "Contractible Side Portions For Disposable Diaper" which issued to K. B. Buell on January 14, 1975.

In the preferred embodiment illustrated in Figure 2 the flaps 37 are unitary with the outer covering layer 11. More specifically, the backsheet portion 16 is sufficiently wide so that the material used for the backsheet portion 16 extends beyond the longitudinal seams 32. In this embodiment, the material used for the backsheet portion 16 is the same as that used for the flaps 37 with the edge of the backsheet portion material adjacent the longitudinal seams 32 forming the fixed edge 40 of the flaps 37.

The liquid contacting surface 43 of each flap 37 is provided with leakage resistant portion 48 (Figure 3). The leakage resistant portion 48 is a portion of the flaps 37 positioned between the distal edge 42 and the fixed edge 40 of the flaps 37. The leakage resistant portion 48 retards the movement of liquid from the fixed edge 40 of the flaps 37 to the distal edge 42 of the flaps 37. The leakage resistant portion 48 thereby provides an obstacle in the path followed by liquid as it tends to move across the liquid contacting surface 43 from the point of discharge toward the distal edge 42 from which liquid leakage can occur. Accordingly, the leakage resistant portion 48 is non-wicking (i.e., liquid contacting the leakage resistant portion 48 will not cross the leakage resistant portion 48 due to capillary liquid transport), non-absorbent (i.e., liquid is not retained by the leakage resistant portion 48 and is therefore free to flow back toward the absorbent core 14), and liquid impermeable. Further, the leakage resistant portion 48 is positioned and dimensioned such that liquid will not pass the leakage resistant portion 48 in normal use of the diaper 10.

In the preferred embodiment illustrated in Figures 2 and 3 the leakage resistant portion 48 is a strip of polyethylene film of the same type as used for the backsheet portion 16. Thus, the leakage resistant portion 48 will neither absorb, nor wick liquid. In addition, the leakage resistant portion 48 is impermeable to liquid; thus, liquid will not pass through the thickness of the leakage resistant portion 48 under normal usage and pressures.

Preferably, the leakage resistant portion 48 has a length which extends the entire length of the flaps 37 and a width of at least 0.125 inches (3 mm). Other dimensions may, however, also be used. For example, the leakage resistant portion 48 may have a length different from the length of the flaps 37. In addition, a variety of widths may be used for the leakage resistant portion 48. While the width of the leakage resistant portion 48 is preferably at least 0.125 inches (3 mm), it is more preferably at least 0.25 inches (6 mm) and still more preferably at least 0.50 inches (12 mm). The larger the width of the leakage resistant portion 48 the less likely liquid is to bridge the leakage resistant portion 48 and thus leak out of the diaper 10.

In use, the diaper 10 is placed between the legs of the diaper wearer and fastened about the wearer's waist using any suitable means such as adhesive tapes 56 as is well known. When the diaper 10 of the present invention is applied to a wearer, it exhibits improved liquid containment characteristics. While not wishing to be bound by any one theory it is believed that the improved liquid containment characteristics are achieved in the following manner.

As urine is discharged onto the topsheet portion 12 some of the urine penetrates the topsheet portion 12 and is absorbed by the absorbent core 14 (hereinafter referred to as absorbed urine), some of the urine flows on the surface of the topsheet portion 12 (hereinafter referred to as surface urine), some of the urine is absorbed by and wicks laterally through the topsheet portion 12 and some of urine flows into the capillary channel formed at the interface between the topsheet portion 12 and the skin of the diaper wearer.

The absorbed urine migrates throughout the absorbent core 14 moving from the point of discharge (i.e., the crotch area 13) toward the peripheral edge 50 of the absorbent core 14. Eventually, the urine will reach the first and second longitudinal side edges 52 and 54 respectively. Since the absorbed urine which encounters the leakage resistant portion 48 is not absorbed by and cannot wick into the flaps 37 and absorbed urine is effectively prevented from leaking out of the diaper 10 at the longitudinal side edges 52 and 54 respectively.

The surface urine, likewise, moves from the point of discharge toward the first and second longitudinal side edges 52 and 54 respectively. Surface urine which contacts the leakage resistant portions 48 is not absorbed. In normal use the gravitational forces will tend to cause the surface urine to drain back toward the absorbent core 14. The surface urine which does cross the leakage resistant portion is retarded from leaking out of the diaper by the sealing effect achieved by the elastic member 44 as its draws the flaps 37 about the legs of the diaper wearer.

The distal edge 42 of the flaps 37 is elastically contractible so that when the diaper 10 is worn the distal edge 42 is drawn about the wearer's legs. The fixed edge 40 normally sags away from the wearer's legs thereby forming a gap between the leakage resistant portion 48 and the skin of the wearer. Liquid flowing along the capillary channel formed between the topsheet portion 12 and the skin of the diaper wearer is prevented from bridging the leakage resistant portion 48 by the discontinuity in the capillary channel at the leakage resistant portion 48. In this manner, liquid is prevented from leaking from the diaper 10.

Finally, urine which is absorbed interstitially by the topsheet portion 12 wicks laterally through the topsheet portion 12 towards first and second longitudinal side edges 52 and 54. As in the instance of the surface urine, the urine absorbed by the topsheet portion 12 encounters the leakage resistant portion 48 which is non-wicking. The urine absorbed by the topsheet portion is thereby prevented from reaching the distal edge 42 from which leakage can occur.

It will be understood by those skilled in the art that the present invention has been described with reference to exemplary embodiments and that variations or modifications can be effected in the described embodiments without departing from the spirit and scope of the invention.

As seen in Figure 4, for example, the flaps 37 may each be utilized as intermediate members 62, as hereinbefore described, to indirectly join the topsheet portion 12 and the backsheet portion 16. Figures 4, 5, and 6 are cross-sectional views of alternatively preferred embodiments of the present invention taken along lines corresponding to line 3—3 of Figure 2. While only one edge of the diaper is shown in and described with reference to Figures 4, 5 and 6 is should be understood that the edges of the diaper 10 are essentially mirror images of each other.

With reference to Figure 4 it can be seen that the flaps 37 are formed by extending the material used for the backsheet portion 16 beyond the first longitudinal side edge 52 of the absorbent core 14 and affixing the edge 64 of the material used for the backsheet portion 16 to the topsheet portion 12. The edge 64 thus coincides with the fixed edge 40. The elastic element 44 if secured to the distal edge 42 and the liquid contacting surface 43 is positioned between the fixed and distal edges 40 and 42.

The leakage resistant portion 48 of the liquid contacting surface 43 is positioned between the distal edge 42 and the fixed edge 40. Since the entire flap 37 of the alternatively preferred embodiment of Figure 4 is manufactured from the same material used for the backsheet portion 16, the leakage resistant member 48 extends from the fixed edge 40 to the distal edge 42.

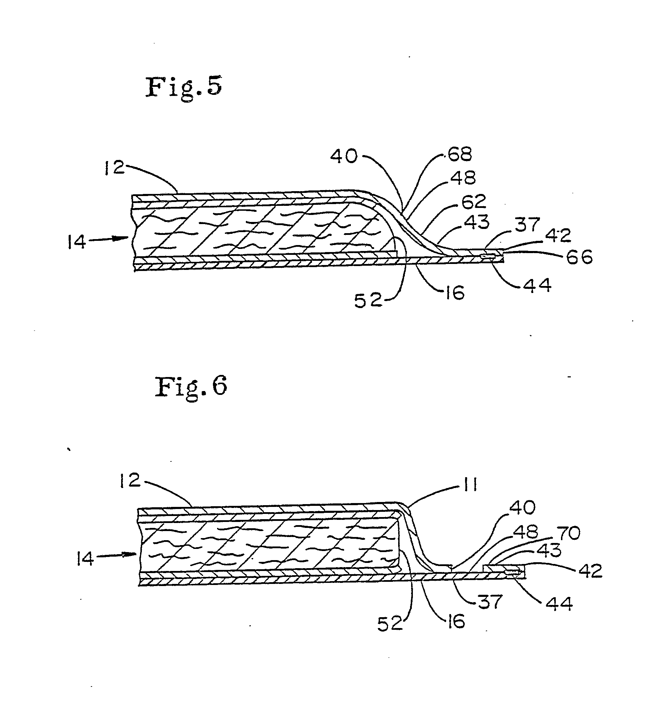

Figure 5 shows an alternatively preferred embodiment in which the flaps 37 are utilized as an intermediate member 62 to indirectly join the topsheet portion 12 and the backsheet portion 16. The flaps 37 are formed by extending the material used for the topsheet portion 12 beyond the first longitudinal side edge 52 of the absorbent core 14 and affixing the edge 66 of the material used for the topsheet portion 12 to the backsheet portion 16.

The marginal portion 68 of the material used for the topsheet portion 12 is treated so as to be liquid impermeable, non-absorbent, and non-wicking. While such treatments are well known in the art a suitable treatment is obtained by applying a food grade paraffin wax such as is marketed by Boron Oil Co., Cleveland, Ohio, under the tradename Boron Wax.

The treated marginal portion 68 forms the leakage resistant portion 48 of the liquid contacting surface 43. As can be seen in Figure 5 the fixed edge 40 coincides with the inward edge of the marginal portion 68 while the distal edge 42 is spaced therefrom. The elastic element 44 is affixed to the flaps 37 at the distal edge 42.

Figure 6 shows an alternatively preferred embodiment in which the flaps 37 are provided with a facing sheet 70. In this embodiment, the material used for this backsheet portion 16 extends beyond the first longitudinal side edge 52 of the absorbent core 14 to form flaps 37 which are unitary with the backsheet portion 16. The topsheet portion 12 is directly joined to the backsheet portion.

The fixed edge 40 of the flaps 37 is connected to the outer covering layer 11 and the elastic element 44 is affixed at the distal edge 42. To prevent direct contact between the elastic element 44 and the skin on the wearer a facing sheet 70 is affixed to the distal edge 42. The facing sheet 70 may be of any flexible material which is non irritating to the skin. In the preferred embodiment illustrated the facing sheet 70 was manufactured from the same material as was used for the topsheet portion 12.

The leakage resistant portion 48 of the liquid contacting surface 43 is positioned between the fixed edge 40 and the distal edge 42.

## Claims

1. A disposable diaper (10) comprising:
an outer covering layer (11) comprising a topsheet portion (12) and a backsheet portion (16), said top sheet portion being joined together with said backsheet portion;
an absorbent core means (14, 24, 26, 28) for absorbing liquid, said absorbent core means having a peripheral edge (50) comprising a first longitudinal side edge (52) and a second longitudinal side edge (54);
a multiplicity of flaps (37, 38, 39), each of said flaps having a fixed edge (40), a distal edge (42) in spaced relation to said fixed edge (40), and having a liquid contacting surface (43), the longitudinal sides of which are bounded by said fixed and said distal edges, one of said flaps (38) having said fixed edge (40) connected to said outer covering layer (11) at said first longitudinal side edge (52) of said absorbent core means (14) and one of said flaps (39) having said fixed edge (40) connected to said outer covering layer (11) at said second longitudinal side edge (54) the distal edge (42) of each of said flaps (38, 39) being displaced from said absorbent core means; and an elastic element (44) affixed to each of said flaps (38, 39),

characterised in that said elastic element (44) is operatively associated with the distal edge (42) of each of said flaps (38, 39) and in that each of said flaps has a leakage resistant portion (48) on said liquid contacting surface (43) interposed between said fixed edge (40) and said distal edge (42), said leakage resistant portion being non-absorbent, and liquid impermeable.

2. A disposable diaper according to claim 1 wherein said leakage resistant portion has a width of at least 3 mm.

3. A disposable diaper according to either one of claims 1 and 2 wherein said leakage resistant portion has a width of at least 6 mm, preferably at least 12 mm.

4. A disposable diaper according to any one of claims 1—3 wherein said distal edge is displaced from said absorbent core means a distance of at least 6 mm.

5. A disposable diaper according to any one of claims 1—4, wherein said distal edge is displaced from said absorbent core means a distance of at least 12 mm, preferably at least 19 mm.

6. A disposable diaper according to any one of claims 1—5 wherein said flaps (38, 39) are unitary with said backsheet portion (16).

7. A disposable diaper according to any one of claims 1—5 wherein said topsheet portion (12) and said backsheet portion (16) are affixed to an intermediate member (62) serving as said leakage resistant portion (48).

8. A disposable diaper according to claim 7 wherein said flaps (37) are utilized as said intermediate members (62).

9. A disposable diaper according to either one of claims 7 and 8 wherein the material used for said topsheet portion (12) has a marginal portion (68) which is liquid impermeable, non-absorbent, and non-wicking and forms said leakage resistant portion (48).

10. A disposable diaper according to any one of claims 1—5 wherein a facing sheet (70) is affixed to said distal edge (42).

**Patentansprüche**

1. Eine Wegwerfwindel (10), umfassend:
eine äußere Abdeckschicht (11) mit einem Abdecklagenteil (12) und einem Unterlagenteil (16), wobei der genannte Abdecklagenteil mit dem genannten Unterlagenteil verbunden ist;
ein saugfähiges Kernelement (14, 24, 26, 28) zum Absorbieren von Flüssigkeit, wobei das genannte saugfähige Kernelement eine Randkante (50) mit einer ersten, in der Längsrichtung verlaufenden Seitenkante (52) und einer zweiten, in der Längsrichtung verlaufenden Seitenkante (54) aufweist;
eine Mannigfaltigkeit von Ansätzen (37, 38, 39), wobei jeder dieser Ansätze eine feststehende Kante (40), eine Distalkante (42), die sich im Abstand von der genannten feststehenden Kante (40) befindet, und eine mit der Flüssigkeit in Berührung kommende Oberfläche (43) aufweist, deren in der Längsrichtung verlaufende Seiten von der genannten feststehenden Kante und von der genannten Distalkante begrenzt sind, wobei die genannte feststehende Kante (40) von einem der genannten Ansätze (38) mit der genannten äußeren Abdeckschicht (11) an der genannten ersten, in der Längsrichtung verlaufenden Seitenkante (52) des genannten saugfähigen Kernelementes (14) verbunden ist, die genannte feststehende Kante (40) von einem der genannten Ansätze (39) mit der genannten äußeren Abdeckschicht (11) an der genannten zweiten, in der Längsrichtung verlaufenden Seitenkante verbunden ist, und die Distalkante (42) von jedem der genannten Ansätze (38, 39) von dem genannten saugfähigen Kernelement abgesetzt ist;
und ein elastisches Element (44), welches an jedem der genannten Ansätze (38, 39) befestigt ist,
dadurch gekennzeichnet, daß das genannten elastische Element (44) in wirkender Verbindung mit der Distalkante (42) von jedem der genannten Ansätze (38, 39) steht, und daß jeder der genannten Ansätze einen gegen Flüssigkeitsaustritt widerstandsfähigen Teil (48) auf der genannten, mit der Flüssigkeit in Berührung kommenden Oberfläche (43) aufweist, der zwischen der genannten feststehenden Kante (40) und der genannten Distalkante (42) augeordnet ist, wobei der genannte, gegen Flüssigkeitsaustritt widerstandfähige Teil nicht-absorbierend und flüssigkeitsundurchlässig ist.

2. Eine Wegwerfwindel nach Anspruch 1, worin der genannte, gegen Flüssigkeitsaustritt widerstandsfähige Teil eine Mindestbreite von 3 mm hat.

3. Eine Wegwerfwindel nach Anspruch 1 oder 2, worin der genannte, gegen Flüssigkeitsaustritt widerstandsfähige Teil eine Mindestbreite von 6 mm, vorzugsweise von 12 mm, hat.

4. Eine Wegwerfwindel nach einem der Ansprüche 1 bis 3, worin die genannte Distalkante von dem genannten saugfähigen Kernelement um einen Mindestabstand von 6 mm abgesetzt ist.

5. Eine Wegwerfwindel nach einem der Ansprüche 1 bis 4, worin die genannte Distalkante von dem genannten saugfähigen Kernelement um einen Mindestabstand von 12 mm, vorzugsweise von 19 mm, abgesetzt ist.

6. Eine Wegwerfwindel nach einem der Ansprüche 1 bis 5, worin die genannten Ansätze (38, 39) mit dem genannten Unterlagenteil (16) einheitlich sind.

7. Eine Wegwerfwindel nach einem der Ansprüche 1 bis 5, worin der genannten Abdecklagenteil (12) und der genannte Unterlagenteil (16) an einem Zwischenglied (62), welches als der genannte, gegen Flüssigkeitsaustritt widerstandsfähige Teil (48) dient, befestigt sind.

8. Eine Wegwerfwindel nach Anspruch 7, worin die genannten Ansätze (37) als die genannten Zwischenglieder (62) verwendet werden.

9. Eine Wegwerfwindel nach Anspruch 7 oder 8, worin das für den genannten Abdecklagenteil (12) verwendete Material eine Randteil (68) aufweist, welcher flüssigkeitsundurchlässig, nicht-absorbierend und ohne Dochtwirkung ist, und welcher den genannten, gegen Flüssigkeitsaustritt widerstandsfähigen Teil (48) bildet.

10. Eine Wegwerfwindel nach einem der Ansprüche 1 bis 5, worin an der gennanten Distalkante (42) eine Abdecklage (70) befestigt ist.

**Revendications**

1. Couche à jeter après usage (10) comportant:
Une couche externe formant enveloppe (11) comportant pour partie une feuille supérieure (12) et pour partie un feuille inférieure (16), la feuille supérieure étant réunie avec la feuille inférieure;
Un moyen central absorbant (14, 24, 26, 28) pour absorber les liquides, ce moyen central absorbant ayant un bord externe (50) qui comporte un premier bord dans le sens longitudinal (52) et un second bord dans le sens longitudinal (54);
Plusieurs pattes (37, 38, 39), chacune de ces pattes ayant une extrémité fixée (40), une extrémité distale (42) à une certaine distance de l'extrémité fixée, et ayant une face au contact du liquide, dont les côtés longitudinaux sont reliés par ces extrémités fixées et distale, une de ces pattes (38) ayant la dite extrémité fixée (40) reliée à la couche externe formant enveloppe (11) au niveau du premier bords dans le sens longitudinal (52) du dit moyen central absorbant (14) et une des dites pattes (39) ayant la dite extrémité fixée (40) reliée à la couche externe formant enveloppe (11) au niveau du second bord dans le sens longitudinal (54), l'extrémité distale (42) de chacune des pattes (38, 39) étant èloignée de ce moyen central absorbant, et un élément élastique (44) fixé à chacune des pattes (38, 39) caractérisé en ce que le dit élément élastique (44) est associé de façon fonctionnelle avec l'extrémité distale (42) de chacune des pattes (38, 39) et en ce que chacune des pattes a une partie ne laissant pas passer de liquide (48) sur la dite face au contact du liquide (43) située entre l'extrémité fixée (40) et l'extrémité distale (42), cette partie ne laissant pas passer de liquide étant non absorbante, et imperméable aux liquides.

2. Couche à jeter après usage selon la revendication 7 dans laquelle la partie ne laissant passer de liquide a une largeur d'au moins 3 mm.

3. Couche à jeter après usage selon l'une des revendications 1 ou 2 dans laquelle la partie ne laissant pas passer de liquide a une largeur d'au moins 6 mm de préférence au moins 12 mm.

4. Couche à jeter après usage selon l'une des revendications 1 à 3 dans laquelle l'extrémité distale est éloignée du dit moyen central absorbant d'au moins 6 mm.

5. Couche à jeter après usage selon l'une quelconque des revendications 1 à 4 dans laquelle l'extrémité distale est éloignée du dit moyen central absorbant d'au moins 12 mm, de préférence d'au moins 19 mm.

6. Couche à jeter après usage selon l'une quelconque des revendications 1 à 5 dans laquelle les pattes (38, 39) forment un tout avec la dite feuille inférieure (16).

7. Couche à jeter après usage selon l'une quelconque des revendications 1 à 5 dans laquelle la dite feuille supérieure (12) et la dite feuille inférieure (16) sont fixées à un élément intermédiaire (62) utilisé comme partie ne laissant pas passer de liquide (48).

8. Couche à jeter après usage selon la revendication 7 dans laquelle les pattes (37) sont utilisées comme éléments intermédiaires (62).

9. Couche à jeter après usage selon l'une des revendications 7 à 8 dans laquelle le matériau utilisé pour la feuille supérieure (12) a une zone marginale (68) imperméable aux liquides, non absorbante, et sans effet de mèche et forme la dite partie ne laissant pas passer de liquide (48).

10. Couche à jeter après usage selon l'une quelconque des revendications 1 à 5 dans laquelle une feuille formant couche extérieure est fixée à l'extrémité distale (42).

Fig. 1

56

17

13

56

10

15

Fig. 2

10

39

42

34

37

37

3

30

12

42

38

42

44

26

20

30

54

40

40

43

32

28

22

52

34

24

11

44

50

50

16

14

32

50

0 070 584

Fig.3

Fig. 4

Fig.5

Fig.6